(19) Europäisches Patentamt
European Patent Office
Office européen des brevets

(11) EP 1 300 145 A1

(12) EUROPEAN PATENT APPLICATION
published in accordance with Art. 158(3) EPC

(43) Date of publication:
**09.04.2003 Bulletin 2003/15**

(21) Application number: **01947800.7**

(22) Date of filing: **04.07.2001**

(51) Int Cl.[7]: **A61K 31/4745**, A61K 31/4985, C07D 471/04, A61P 25/24, A61P 25/22, A61P 25/18

(86) International application number:
**PCT/JP01/05779**

(87) International publication number:
**WO 02/002117 (10.01.2002 Gazette 2002/02)**

(84) Designated Contracting States:
**AT BE CH CY DE DK ES FI FR GB GR IE IT LI LU MC NL PT SE TR**
Designated Extension States:
**AL LT LV MK RO SI**

(30) Priority: **04.07.2000 JP 2000201865**

(71) Applicant: **TORAY INDUSTRIES, INC.
Tokyo 103-8666 (JP)**

(72) Inventors:
- **NAGASE, Hiroshi
Kamakura-shi, Kanagawa 247-0063 (JP)**
- **TANAKA, Toshiaki
Zushi-shi, Kanagawa 249-0004 (JP)**
- **SAITOH, Akiyoshi
Kanagawa 248-0031 (JP)**

(74) Representative: **Kador & Partner
Corneliusstrasse 15
80469 München (DE)**

# (54) THERAPEUTIC OR PREVENTIVE MEDICINES FOR MOOD DISORDERS OR ANXIETY DISORDERS

(57) A drug for curing or preventing mood disorder or anxiety disorder that has fewer and milder side effects comprising, as an effective component, an isoquinoline derivative or a physiologically acceptable acid addition salt thereof, the representative example of the isoquinoline derivative being (4aS, 12aS)-2-methyl-4a-(3-hydroxyphenyl)-1,2,3,4,4a,5,12,12a-octahydroquinolino [2,3-g]isoquinoline; a method for curing or preventing mood disorder or anxiety disorder using the same; and a use of the isoquinoline derivative or the physiologically acceptable acid addition salt thereof for the manufacture of a medicament for curing or preventing mood disorder and anxiety disorder.

EP 1 300 145 A1

## Description

Technical Field

The present invention relates to a drug for curing or preventing mood disorder or anxiety disorder comprising an isoquinoline derivative or a physiologically acceptable acid addition salt thereof, to a method for curing or preventing mood disorder or anxiety disorder using the same, and to a use of the isoquinoline derivative or the physiologically acceptable acid addition salt thereof for the manufacture of a medicament for curing or preventing mood disorder or anxiety disorder.

Background Art

In modern aging societies and high stress societies, psychiatric disorders have shown an increase. Particularly, mood disorder and anxiety disorder have shown a sharp increase.

Tricyclic antidepressants are used as the therapeutic drug for curing mood disorder. Representative examples thereof are imipramine and desipramine. However, tricyclic antidepressants have many side effects and thus there is a problem of tolerance. For example, tricyclic antidepressants require several weeks before their therapeutic effects. appear, are cardiotoxic if overdosed, and have various side effects such as dry mouth, constipation, and difficulty urinating. These side effects cause a decrease in tolerance. Recently, selective serotonin reuptake inhibitors (SSRIs), such as fluvoxamine and fluoxetine, have been developed as mood disorder therapeutic drugs that have fewer and milder side effects than tricyclic antidepressants. SSRIs are safer than the existing drugs and are superior in that SSRIs are effective against newer adaptive disorders such as obsessive compulsive disorder and panic disorder. However, the side effects including digestive disorders such as nausea, vomiting, and diarrhea, sexual dysfunction, headache, and insomnia are still reported. Moreover, SSRIs require several weeks before their therapeutic effects appear and have a low effectiveness against patients of severe depression. Development of therapeutic drugs for mood disorder that have fewer and milder side effects and can reliably achieve the effect is strongly desired.

Benzodiazepine anxiolytics are used as the therapeutic drugs for anxiety disorder. The representative example thereof is diazepam. However, the drug has excessive sedative effects including drowsiness and dizziness, and induces side effects such as mental and physical dependence, memory disorder, and muscle relaxation, thereby often obstructing the everyday life of patients. Particularly, the side effect of mental and physical dependence due to chronic administration has resulted in abuse, which is a serious problem. In practice, the drug administration must be optimized individually for each patient, and thus the use of this therapeutic drug is difficult. As therapeutic drugs for anxiety disorder that have fewer and milder side effects than the benzodiazepine anxiolytics, serotonergic anxiolytics have been developed. The representative example thereof is buspirone. The advantages of buspirone include milder sedative effects, low physical dependence, and less effect on mental activities when taken with ethanol. However, buspirone requires long time before the therapeutic effects to appear compared to benzodiazepine anxiolytics that work fast. Moreover, the effectiveness for patients of severe anxiety disorder is low. Thus, development of therapeutic drugs for anxiety disorder that have fewer and milder side effects and can reliably achieve the effect is strongly desired.

Examples or prior art related to isoquinoline derivatives include Japanese Unexamined Patent Application Publication No. 4-275288, WO 93/01186, and WO 99/02157. However, these documents only disclose immunosuppressants, analgesics, and antitussives and do not teach its use as a drug for curing or preventing psychiatric disorders.

The present invention aims to provide a drug for curing or preventing mood disorder or anxiety disorder that have fewer and milder side effects and can reliably achieve its effect and a method for curing or preventing mood or anxiety disorders.

Disclosure of Invention

The present invention includes a drug for curing or preventing mood disorder or anxiety disorder comprising an isoquinoline derivative represented by formula (I) below or a physiologically acceptable acid addition salt thereof; a method for curing or preventing mood disorder or anxiety disorder using the same; and a use of the isoquinoline derivative or the physiologically acceptable acid addition salt thereof for the manufacture of a medicament for curing or preventing mood disorder or anxiety disorder:

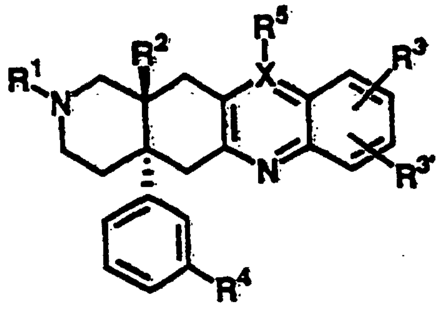

(I)

wherein $R^1$ is hydrogen, $C_1$-$C_5$ alkyl, $C_4$-$C_7$ cycloalkylalkyl, $C_5$-$C_7$ cycloalkenylalkyl, $C_7$-$C_{14}$ aralkyl, $C_4$-$C_5$ trans-alkenyl, allyl, furan-2-ylalkyl($C_1$-$C_5$), thien-2-ylalkyl($C_1$-$C_5$), $C_1$-$C_5$ alkanoyl, benzoyl, vinyloxycarbonyl, trichloroethoxycarbonyl, benzyloxycarbonyl, or $C_8$-$C_{14}$ arylalkanoyl; $R^2$ is hydrogen or $OR^6$ (wherein $R^6$ is hydrogen, $C_1$-$C_5$ alkyl, or $C_1$-$C_5$ alkanoyl); $R^3$ and $R^{3'}$ are, independently, $C_1$-$C_5$ alkyl, hydrogen, chlorine, fluorine, bromine, iodine, trifluoromethyl, cyano, hydroxy, $C_1$-$C_3$ alkoxycarbonyl, $C_1$-$C_3$ alkylcarbonylamino, $C_1$-$C_5$ alkoxy, nitro, amino, $C_1$-$C_3$ alkylamino, or $C_2$-$C_6$ dialkylamino; $R^4$ is hydrogen, hydroxy, $C_1$-$C_3$ alkoxy, benzyloxy, $C_1$-$C_5$ alkanoyloxy, chlorine, fluorine, bromine, or iodine; X is nitrogen or carbon; $R^5$ exists only when X is carbon and is $C_1$-$C_5$ alkyl, hydrogen, chlorine, fluorine, bromine, iodine, trifluoromethyl, cyano, hydroxy, $C_1$-$C_3$ alkoxycarbonyl, $C_1$-$C_3$ alkylcarbonylamino, $C_1$-$C_5$ alkoxy, nitro, amino, $C_1$-$C_3$ alkylamino, or $C_2$-$C_6$ dialkylamino.

Brief Description of the Drawings

Fig. 1 is a graph showing the antianxiety effect of Compound 1 in an elevated plus-maze test with rats.
Fig. 2 is a graph showing antidepressant effect of Compound 1 in a forced swim test with mice.

Best Mode for Carrying Out the Invention

In an isoquinoline derivative represented by formula (I), $R^1$ is preferably, hydrogen, $C_1$-$C_5$ alkyl, $C_4$-$C_7$ cycloalkylalkyl, $C_5$-$C_7$ cycloalkenylalkyl, $C_7$-$C_{14}$ aralkyl, $C_4$-$C_5$ trans-alkenyl, allyl, furan-2-ylalkyl ($C_1$-$C_5$), or thien-2-ylalkyl ($C_1$-$C_5$). More preferably, $R^1$ is hydrogen, methyl, ethyl, cyclopropylmethyl, allyl, phenethyl, furan-2-ylethyl, or thiophen-2-ylethyl.

Preferably, $R^2$ is hydrogen, hydroxy, methoxy, ethoxy, or acetoxy.

Preferably, $R^3$ and $R^{3'}$ are, independently, $C_1$-$C_5$ alkyl, hydrogen, chlorine, fluorine, bromine, iodine, hydroxy, $C_1$-$C_5$ alkoxy, nitro, amino, $C_1$-$C_3$ alkylamino, or $C_2$-$C_6$ dialkylamino. More preferably, $R^3$ and $R^{3'}$ are, independently, methyl, hydrogen, chlorine, fluorine, bromine, iodine, hydroxy, methoxy, nitro, amino, or dimethylamino.

$R^4$ is preferably hydrogen, hydroxy, $C_1$-$C_3$ alkoxy, or $C_1$-$C_5$ alkanoyloxy. More preferably, $R^4$ is hydrogen, hydroxy, methoxy, or acetoxy.

X is preferably carbon. When X is carbon, $R^5$ is preferably $C_1$-$C_5$ alkyl, hydrogen, chlorine, fluorine, bromine, iodine, hydroxy; $C_1$-$C_5$ alkoxy, nitro, amino, $C_1$-$C_3$ alkylamino, or $C_2$-$C_6$ dialkylamino. More preferably, $R^5$ is methyl, hydrogen, chlorine, fluorine, bromine, iodine, hydroxy, methoxy, nitro, amino, or dimethylamino.

Preferable examples of physiologically acceptable acid addition salt include salts of inorganic acids such as hydrochloric acid, sulfuric acid, nitric acid, hydrobromic acid, hydriodic acid, phosphoric acid, salts of organic carboxylic acids such as acetic acid, lactic acid, citric acid, oxalic acid, glutaric acid, malic acid, tartaric acid, fumaric acid, mandelic acid, maleic acid, benzoic acid, and phthalic acid, and salts of organic sulfonic acids such as methanesulfonic acid, ethanesulfonic acid, benzenesulfonic acid, p-toluenesulfonic acid, camphorsulfonic acid. Among them, salts of hydrochloric acid, hydrobromic acid, phosphoric acid, tartaric acid, methanesulfonic acid, and the like are particularly preferred.

The compound represented by formula (I) of the present invention can be prepared by, for example, a method described in Japanese Unexamined Patent Application Publication No. 4-275288 or WO 99/02157 whereby a ketone body (III), i.e., a raw material, is condensed with an o-aminobenzaldehyde derivative (IVa), an o-aminoacetophenone derivative (IVb), or an o-aminobenzonitrile. (IVc) in a solvent in the presence of an acid catalyst. When an optically active substance is used as the raw ingredient, an optically active compound can be obtained (Scheme 1).

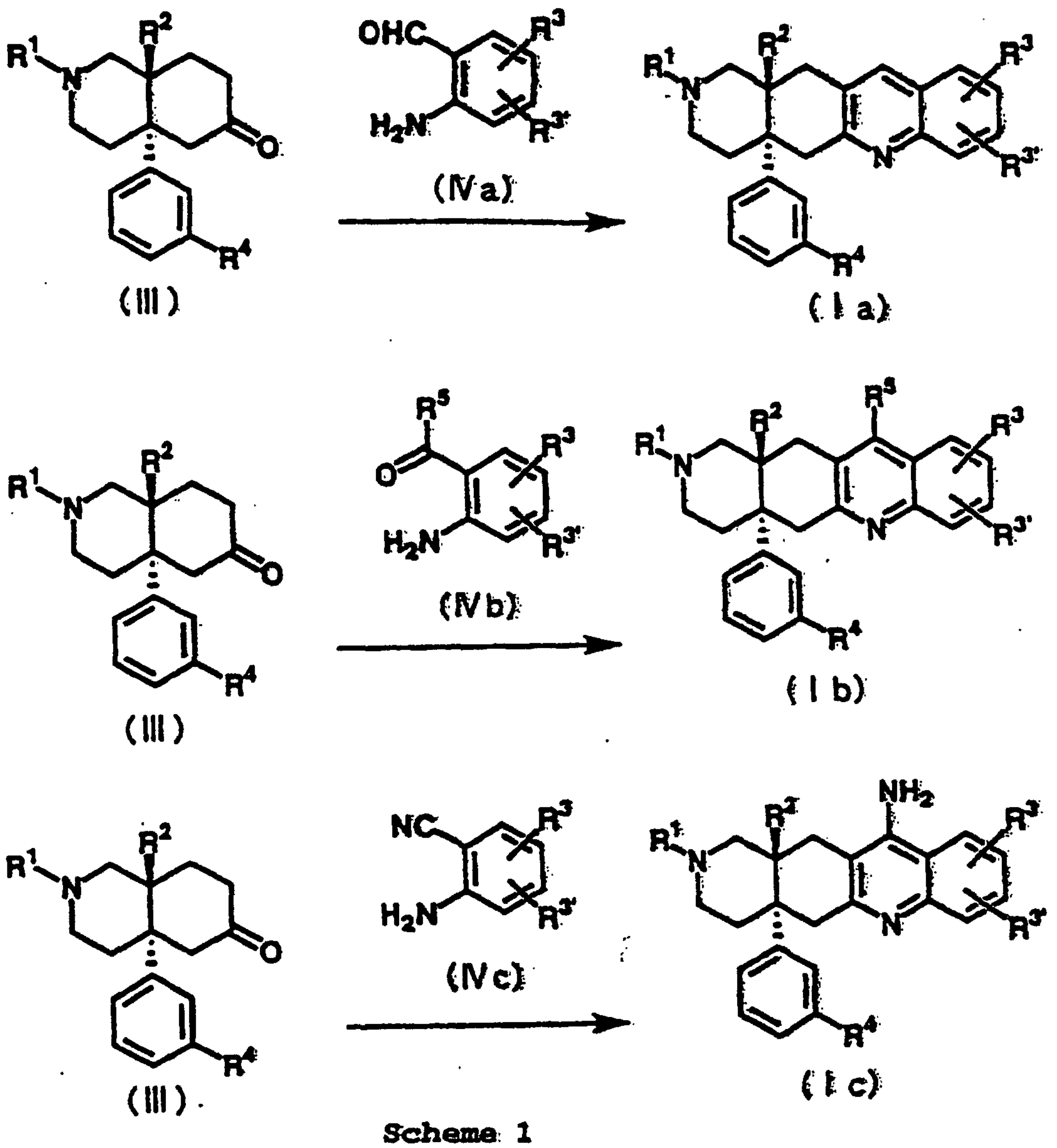

(III) (IVa) (Ia)

(III) (IVb) (Ib)

(III) (IVc) (Ic)

Scheme 1

The isoquinoline derivative represented by formula (I) has a marked effect in the forced swim test, which is the animal model for mood disorder, and in the elevated plus-maze test, which is the animal model for anxiety disorder. Thus, the isoquinoline derivative can be used as a drug for curing or preventing mood disorder or anxiety disorder of mammal and, in particular, human beings. It may also be used in manufacturing of a medicament for curing or preventing mood disorder or anxiety disorder. The evaluation using the above-described animal models can be conducted by a method described in published references [Nature vol. 266, 730 (1977) or Jpn. J. Psychopharmacol vol. 15, 125 (1995)], but the method is not limited to this.

Herein, the term "mood disorder" refers to the state of uncontrollable mood or emotion. More specifically, the term refers to the disorders such as major depression disorder, bipolar disorder, dysthymic disorder, and cyclothymic disorder. Depression disorder due to the withdrawal from dependence-producing drugs and cognitive impairment due to aging are also included in the mood disorder.

On the other hand, the anxiety disorder refers to the mental state that possibly obstructs the daily lives of the patients due to anxiety. Specific examples thereof include panic disorder, agoraphobia, social phobia, obsessive-compulsive disorder, posttraumatic stress disorder, acute stress disorder, and generalized anxiety disorder. Note that the application of the drug of the present invention for curing or preventing mood disorder or anxiety disorder is not limited to the above-described examples.

In the clinical use of the drug of the present invention for curing or preventing mood disorder or anxiety disorder, the drug may be a free base or a salt itself, and may adequately contain additives such as a filler, a stabilizer, a

preservative, a buffer, a solubilizer, an emulsifier, a thinner, and an isotonizing agent. Either parenteral administration or oral administration can be employed. Examples of dosage forms of administration include injection, tablets, liquid, capsules, granules, and powder, and the drug in the above-described forms may be manufactured by known drug-making processes. The dosage is suitably selected according to the symptom, age, and weight of the patient, and the route of administration. The effective amount for an adult daily is 0.0001 mg to 10 g, and preferably, 0.001 mg to 1 g. This amount can be administered once or over several doses.

[Examples]

The present invention will now be described in the following reference and examples.

Reference 1

Preparation of methanesulfate salt of (4aS,12aS)-2-methyl-4a-(3-hydroxyphenyl)-1,2,3,4,4a,5,12,12a-octahydroquinolino[2,3-g]isoquinoline (Compound 1)

The compound was prepared by a method described in Japanese Unexamined Patent Application Publication No. 4-275288.

Reference 2

2-methyl-4aα-(3-methoxyphenyl)-11-methyl-1,2,3,4,4a,5,12,12aβ-octahydro-quinolino[2,3-g]isoquinoline (Compound 2)

To 5 mL of acetic acid, 150 mg (0.55 mmol) of 2-methyl-4aα-(3-methoxyphenyl)-6-oxo-1,2,3,4,4a,5,6,7,8,8aβ-octahydroisoquinoline and 100 mg (0.74 mmol) of o-aminoacetophenone were added, and the resulting mixture was heated for three hours under reflux. After spontaneous cooling, the mixture was added with a saturated sodium hydrogen carbonate aqueous solution, and extraction with ethyl acetate was performed. The organic layer was washed with saturated brine, dried with anhydrous magnesium sulfate, and condensed. The resulting residue was subjected to separation and refinement by silica gel column chromatography (chloroform:methanol:aqueous ammonia = 20:1:0.1 to 10:1:0.1) to obtain 211 mg of title compound (100% yield).

Reference 3

2-methyl-4aα-(3-hydroxyphenyl)-11-methyl-1,2,3,4,4a,5,12,12aβ-octahydro-quinolino[2,3-g]isoquinoline (Compound 3) as a hydrochloride salt

In an argon atmosphere, 210 mg (0.56 mmol) of 2-methyl-4aα-(3-methoxyphenyl)-11-methyl-1,2,3,4,4a,5,12,12aβ-octahydro-quinolino[2,3-g]isoquinoline obtained in Reference 2 and 0. 29 mL (3.20 mmol) of n-propanethiol were dissolved in 7 mL of a DMF solvent, and 320 mg (2.85 mmol) of pottasium-t-butoxide was added. The resulting mixture was heated at 120°C for 20 hours while being stirred. To the resulting mixture in an ice-water bath, 4 mL of 1-normal hydrochloric acid was added to make the mixture acidic. Subsequently, a saturated sodium hydrogen carbonate aqueous solution was added to make the resulting mixture alkaline again, and extraction with a chloroform: methanol (4:1) mixed solvent was performed. The organic layer was washed with water, dried with anhydrous magnesium sulfate, and condensed. The resulting residue was recrystallized using a dichloromethane-methanol mixed solvent so as to obtain 131 mg of title compound (65% yield). The compound was salified with methanol/hydrochloric acid. After condensation, ether was added, and the solid was separated by filtering so as to obtain 143 mg of hydrochloride salt of the title compound.

Reference 4

2-methyl-4aα-(3-methoxyphenyl)-11-amino-1,2,3,4,4a,5,12,12aβ-octahydro-quinolino[2,3-g]isoquinoline (Compound 4)

To 5 mL of acetic acid, 150 mg (0.55 mmol) of 2-methyl-4aα-(3-methoxyphenyl)-6-oxo-1,2,3,4,4a,5,6,7,8,8aβ-octahydroisoquinoline and 130 mg (1.10 mmol) of o-aminobenzonitrile were added, and the resulting mixture was heated for 44 hours under reflux. After spontaneous cooling, the mixture was added with a saturated sodium hydrogen carbonate aqueous solution, and extraction with a chloroform:methanol (4:1) mixed solvent was performed. The organic

layer was washed with saturated brine, dried with anhydrous magnesium sulfate, and condensed. The resulting residue was subjected to separation and refinement by amine-coated silica gel column chromatography (chloroform:methanol = 50:1) to obtain 83 mg of title compound (41% yield).

Reference 5

2-methyl-4aα-(3-hydroxyphenyl)-11-amino-1,2,3,4,4a,5,12,12aβ-octahydro-quinolino[2,3-g]isoquinoline (Compound 5) as a methanesulfonate salt

In an argon atmosphere, 83 mg (0.22 mmol) of 2-methyl-4aα-(3-methoxyphenyl)-11-amino-1,2,3,4,4a,5,12,12aβ-octahydro-quinolino[2,3-g]isoquinoline obtained in Reference 4 and 0.10 mL (1.06 mmol) of n-propanethiol were dissolved in 7 mL of a DMF solvent, and 106 mg (0.95 mmol) of pottasium-t-butoxide was added to the resulting mixture. The resulting mixture was heated at 120°C for 20 hours while being stirred. To the mixture in an ice-water bath, 4 mL of 1-normal hydrochloric acid was added to make the mixture acidic, and a saturated sodium hydrogen carbonate aqueous solution was then added to the mixture so as to make the mixture alkaline again. Extraction with a chloroform:methanol (4:1) mixed solvent was performed. The organic layer was washed with water, dried with anhydrous magnesium sulfate, and condensed. The resulting residue was recrystallized using a dichloromethane-methanol mixed solvent so as to obtain 35 mg of title compound (44% yield). The compound was suspended in methanol, salified by adding methanesulfonic acid, and condensed. Subsequently, ether was added and solids were separated by filtering so as to obtain 40 mg of methanesulfonate salt of the title compound.

Reference 6

2-phenethyl-4aα-(3-hydroxyphenyl)-1,2,3,4,4a,5,12,12aβ-octahydro-quinolino [2,3-g]isoquinoline (Compound 6) as methanesulfonate salt

To 8 mL of ethanol, 248 mg (0.71 mmol) of 2-phenethyl-4aα-(3-hydroxyphenyl)-6-oxo-1,2,3,4,4a,5,6,7,8,8aβ-octahydroisoquinoline, 431 mg (0.3.56 mmol) of o-aminobenzaldehyde, and 0.22 ml (3.40 mmol) of methanesulfonic acid were added, and the mixture was heated for 3 hours under reflux. After spontaneous cooling, the mixture was added with a saturated sodium hydrogen carbonate aqueous solution, and extraction with a chloroform:methanol (5:1) mixed solvent was performed. The organic layer was washed with water, dried with anhydrous magnesium sulfate, and condensed. The resulting residue was recrystallized from a dichloroethane-methanol mixed solvent to obtain 245 mg of title compound (80% yield). The compound was suspended in methanol, was salified by adding methanesulfonic acid, and was condensed. Subsequently, ethyl acetate was added, solids were separated by filtering, and 318 mg of methanesulfonate salt of the title compound was obtained.

The structure formulae, the acid addition salts, and various spectrum data of Compounds 2, 3, 4, 5, and 6 described in the corresponding Reference are shown in Table 1.

Table 1

| | | |
|---|---|---|
| Compound 2<br>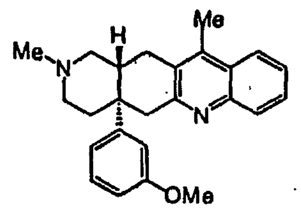 | NMR (ppm) (300 MHz, CDCl3)<br>2.0 (1H, m), 2.16 (1H, m), 2.28 (1H, m), 2.40 (3H, s), 2.51 (3H, s), 2.6-2.8 (2H, m), 2.82 (1H, t, J=11.5Hz), 3.0-3.1 (3H, m), 3.13 (1H, d, J=16.5Hz), 3.68 (3H, s), 3.72 (1H, d, J=16.5Hz), 6.58 (1H, m), 7.05 (3H, m), 7.42 (1H, dt, J=7.1, 1.4Hz), 7.56 (1H, dt, J=7.1, 1.4Hz), 7.9 (2H, m) | Melting Point (℃).<br>Elemental analysis<br>Composition formula<br>Calculated value<br>*Observed value*<br>IR (cm$^{-1}$) (KBr)<br>2928, 2838, 1607, 1582, 1487, 1431, 1288, 1251, 1230, 1048<br>Mass (EI) 372 (M+) |
| Compound 3<br>Hydrochloride salts<br>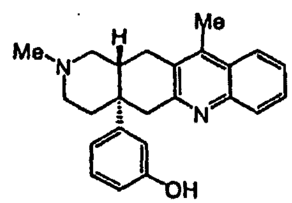 | NMR (ppm) (500 MHz, D2O)<br>2.27 (1H, dt, J=14.3, 3.4Hz), 2.68 (1H, t, J=11.5Hz), 2.81 (3H, s), 2.84 (3H, s), 2.9-3.0 (1H, m), 3.3 (2H, m), 3.4-3.6 (4H, m), 3.68 (1H, dd, J=12.3, 2.8Hz), 3.95 (1H, d, J=17.1Hz), 6.54 (1H, dd, J=8.1, 2.0Hz), 6.96 (1H, d, J=7.9Hz), 7.01 (1H, t, J=2.0Hz), 7.06 (1H, t, J=7.9Hz), 7.83 (1H, t, J=7.9Hz), 7.99 (1H, t, J=7.7Hz), 8.22 (1H, d, J=8.5Hz), 8.39 (1H, d, J=8.7Hz) | Melting Point (℃).<br>Elemental analysis<br>Composition formula C24H26N2O / 2.0HCl / 0.6H2O<br>Calculated value C:65.19, H:6.66, N:6.33, Cl:16.03<br>Observed value C:65.05, H:6.90, N:6.31, Cl:16.13<br>IR (cm$^{-1}$)<br>Mass (EI) 358 (M+) |

Table 1 (continued)

| | | |
|---|---|---|
| Compound 4 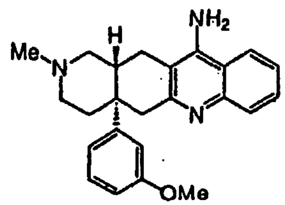 | NMR (ppm) (300 MHz, CDCl3) 2.0 (1H, m), 2.1 (1H, m), 2.28 (1H, m), 2.38 (3H, s), 2.6-2.9 (5H, m), 2.97 (1H, dd, J=11.0, 3.3Hz), 3.05 (1H, d, J=16.5Hz), 3.64 (1H, d, J=16.8Hz), 3.67 (3H, s), 4.58 (2H, brs), 6.58 (1H, m), 7.05 (3H, m), 7.32 (1H, dt, J=7.5, 1.2Hz), 7.51 (1H, dt, J=7.5, 1.2Hz), 7.61 (1H, dd, J=8.5, 0.5Hz), 7.82 (1H, dd, J=8.5, 0.5Hz) | Melting Point (℃).<br>Elemental analysis<br>Composition formula<br>Calculated value<br>Observed value<br>IR ($cm^{-1}$) (KBr)<br>3058, 2918, 2800, 1651, 1578, 1502, 1439, 1243, 1046<br>Mass (EI) 373 (M+) |
| Compound 5 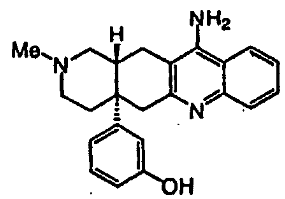 | NMR (ppm) (500 MHz, D2O) 2.21 (1H, dt, J=14.5, 2.5Hz) 2.49 (1H, d, J=12.5Hz), 2.67 (1H, t, J=12.7Hz), 2.84 (3H, s), 2.8-2.9 (3H, m), 3.17 (1H, d, J=16.9Hz), 3.4-3.5 (3H, m), 3.60 (1H, d, J=10.3Hz), 6.58 (2H, m), 6.90 (2H, m), 7.09 (1H, t, J=8.1Hz), 7.61 (1H, d, J=7.3, 1.6Hz), 7.86 (2H, m), 8.47 (1H, d, J=8.5Hz) | Melting Point (℃).<br>Elemental analysis<br>Composition formula<br>Calculated value<br>Observed value<br>IR ($cm^{-1}$)<br>Mass (EI) 359 (M+) |

Table 1 (continued)

| Compound | NMR | Properties |
|---|---|---|
| Compound 6 Methanesulfonate salt 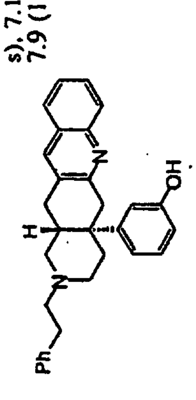 | NMR (ppm) (300 MHz, D2O) 2.2 (1H, m), 2.78 (6H, s), 2.7-3.0 (3H, m), 3.1 (2H, m), 3.4-3.6 (6H, m), 3.68 (1H, t, J=12.9Hz), 3.8-3.9 (2H, m), 6.69 (1H, d, J=7.7Hz), 7.00 (1H, s), 7.1-7.2 (2H, m), 7.3-7.4 (5H, m), 7.7 (1H, m), 7.9 (1H, m), 7.99 (1H, d, J=8.0Hz), 8.70 (1H, s) | Melting Point (℃). <br> Elemental analysis <br> Composition formula: C30H30N2O / 2.08MeSO3H / 0.2H2O <br> Calculated value: C:60.39, H:6.12, N:4.39, S:10.45 <br> Observed value: C:60.47, H:5.90, N:4.45, S:10.42 <br> IR ($cm^{-1}$) <br> Mass |

Example 1

Assessment of antianxiety effect by the elevated plus-maze test in rats

The experiment was conducted on male SD rats. In the elevated plus-maze test, the elevated plus-maze

(manufactured by NeuroScience, Inc.) consisting of closed arms and open arms was used. In the experiment, a rat was placed on an open arm 30 minutes after the drug administration (subcutaneous administration), and the number of entries to the open and closed arms and the time spent in the open arms were measured. The test was conducted for 3 minutes. The rat was assumed to have made entries when all four legs have entered the arm. The ratio of the time spent in the open arms and the ratio of the entries to the open arms were calculated according to the following equations:

$$\text{Ratio of the time spent in the open arms (\%)} = \{(\text{time spent in the open arms})/(\text{total time})\} \times 100$$

$$\text{Ratio of the entries to the open arms (\%)} = \{(\text{number of entries to the open arms})/(\text{total number of entries})\} \times 100$$

The results are shown in Fig. 1. The vertical axis on the left indicates the ratio of the time spent in the open arms (%) and the ratio of the entries to the open arms (%). The vertical axis on the right indicates the total number of entries. The horizontal axis indicates a control group and treatment groups. Open columns indicate the ratio of the time spent in the open arms (%) and hatched columns indicate the ratio of the entries to the open arms (%). Closed circles indicate the total number of entries. All data are indicated by average ±standard error. In a significance test, the difference was assumed to be significant at a significance level of 5% or less by Dunnett's multiple comparison. In the test, the drug is assumed to have an antianxiety effect when there are an increase in time spent on open arms and an increase in ratio of entries to open arms under administration. In Compound 1 treatment groups, a dose-dependent significant increase in both the time spent in open arms and the ratio of the entries to open arms was observed when compared with the control group (saline-treated group). Accordingly, Compound 1 was confirmed to have an antianxiety effect.

Example 2

The antianxiety effect of Compounds 3, 5, and 6 was assessed as in Example 1. The results are shown in Table 2. All showed significant antianxiety effects.

Table 2

| Effect of Compounds 3, 5, and 6 in the elevated plus-maze test in rats | |
|---|---|
| Compound (dose) | Time spent on open arms (sec) |
| Saline,s.c. | 19.1 ± 6.3 |
| 3 (30 mg/kg) | 36.6 ± 13.2 |
| 5 (10 mg/kg) | 25.4 ± 10.2 |
| 6 (30 mg/kg) | 37.9 ± 4.4 |

Example 3

Assessment of antidepressant effect by the forced swim test in mice

In the experiment, male ICR mice were used. In the forced swim test, the tank for forced swim used was a transparent cylindrical tank made of plastic (inner diameter: 10 cm, height: 30 cm), and water at 25°C was contained up to 14 cm in height. A mouse was placed in water, and its swimming time during 2 to 6 minutes after placement was measured. The drug was subcutaneously administered 30 minutes before testing. The results are shown in Fig. 2. The vertical axis indicates the swimming time (sec) during the measurement time (4 min). The open column indicates a saline-treated group and the hatched columns indicate Compound 1 treatment groups. All data are indicated by average ±standard error. In a significance test, the difference was assumed to be significant at a significance level of 5% or less by Dunnett's multiple comparison. In the test, the drug is assumed to have an antidepressant effect when there is an increase in swimming time under administration. In the Compound 1 treatment groups, a dose-dependent significant increase in the swimming time was observed when compared with the control group (saline-treated group). Accordingly, Compound 1 was confirmed to have an antidepressant effect.

Example 4

The antidepressant effect of Compound 3, 5, and 6 was assessed as in Example 3. The results are shown in Table 3. All showed significant antidepressant effect.

Table 3

| Effect of Compounds 3, 5, and 6 in the forced swim test in mice | |
|---|---|
| Compound (dose) | Immobility time (sec) |
| Saline,s.c. | 136.3 ± 11.4 |
| 3 (30 mg/kg) | 75.4 ± 11.6 |
| 5 (10 mg/kg) | 94.3 ± 13 |
| 6 (30 mg/kg) | 58.7 ± 9.1 |

Industrial Applicability

The drug of the present invention is effective as a novel drug for curing or preventing mood disorder or anxiety disorder and has fewer and milder side effects.

## Claims

**1.** A drug for curing or preventing mood disorder or anxiety disorder, comprising an effective amount of an isoquinoline derivative represented by formula (I) or a physiologically acceptable acid addition salt thereof:

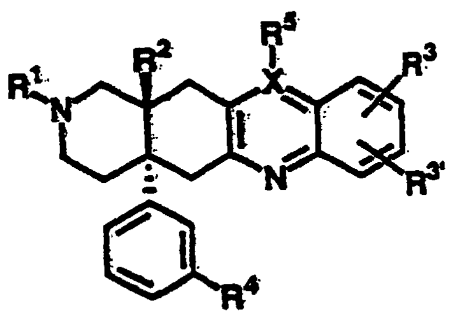

(I)

wherein $R^1$ is hydrogen, $C_1$-$C_5$ alkyl, $C_4$-$C_7$ cycloalkylalkyl, $C_5$-$C_7$ cycloalkenylalkyl, $C_7$-$C_{14}$ aralkyl, $C_4$-$C_5$ trans-alkenyl, allyl, furan-2-ylalkyl ($C_1$-$C_5$), thien-2-ylalkyl ($C_1$-$C_5$), $C_1$-$C_5$ alkanoyl, benzoyl, vinyloxycarbonyl, trichloroethoxycarbonyl, benzyloxycarbonyl, or $C_8$-$C_{14}$ arylalkanoyl; $R^2$ is hydrogen or $OR^6$ (wherein $R^6$ is hydrogen, $C_1$-$C_5$ alkyl, or $C_1$-$C_5$ alkanoyl); $R^3$ and $R^{3'}$ are, independently, $C_1$-$C_5$ alkyl, hydrogen, chlorine, fluorine,. bromine, iodine., trifluoromethyl, cyano, hydroxy, $C_1$-$C_3$ alkoxycarbonyl, $C_1$-$C_3$ alkylcarbonylamino, $C_1$-$C_5$ alkoxy, nitro, amino, $C_1$-$C_3$ alkylamino, or $C_2$-$C_6$ dialkylamino; $R^4$ is hydrogen, hydroxy, $C_1$-$C_3$ alkoxy, benzyloxy, $C_1$-$C_5$ alkanoyloxy, chlorine, fluorine, bromine, or iodine; X is nitrogen or carbon; $R^5$ exists only when X is carbon and is $C_1$-$C_5$ alkyl, hydrogen, chlorine, fluorine, bromine, iodine, trifluoromethyl, cyano, hydroxy, $C_1$-$C_3$ alkoxycarbonyl, $C_1$-$C_3$ alkylcarbonylamino, $C_1$-$C_5$ alkoxy, nitro, amino, $C_1$-$C_3$ alkylamino, or $C_2$-$C_6$ dialkylamino.

**2.** The drug for curing or preventing mood disorder or anxiety disorder according to claim 1, wherein, in formula (I), $R^1$ is hydrogen, $C_1$-$C_5$ alkyl, $C_4$-$C_7$ cycloalkylalkyl, $C_5$-$C_7$ cycloalkenylalkyl, $C_7$-$C_{14}$ aralkyl, $C_4$-$C_5$ trans-alkenyl, allyl, furan-2-ylalkyl ($C_1$-$C_5$) , or thien-2-ylalkyl ($C_1$-$C_5$); $R^3$ and $R^{3'}$ are, independently, $C_1$-$C_5$ alkyl, hydrogen, chlorine, fluorine, bromine, iodine, hydroxy, $C_1$-$C_5$ alkoxy, nitro, amino, $C_1$-$C_3$ alkylamino, or $C_2$-$C_6$ dialkylamino; $R^4$ is hydrogen, hydroxy, $C_1$-$C_3$ alkoxy, or $C_1$-$C_5$ alkanoyloxy; X is carbon, and $R^5$ is $C_1$-$C_5$ alkyl, hydrogen, chlorine,

fluorine, bromine, iodine, hydroxy, $C_1$-$C_5$ alkoxy, nitro, amino, $C_1$-$C_3$ alkylamino, or $C_2$-$C_6$ dialkylamino.

**3.** The drug for curing or preventing mood disorder or anxiety disorder according to claim 1, wherein, in formula (I), $R^1$ is hydrogen, methyl, ethyl, cyclopropylmethyl, allyl, phenethyl, furan-2-ylethyl, or thiophen-2-ylethyl; $R^2$ is hydrogen, hydroxy, methoxy, ethoxy, or acetoxy; $R^3$ and $R^{3'}$ are, independently, methyl, hydrogen, chlorine, fluorine, bromine, iodine, hydroxy, methoxy, nitro, amino, or dimethylamino; $R^4$ is hydrogen, hydroxy, methoxy, or acetoxy; X is carbon; and $R^5$ is methyl, hydrogen, chlorine, fluorine, bromine, iodine, hydroxy, methoxy, nitro, amino, or dimethylamino.

**4.** The drug for curing or preventing mood disorder or anxiety disorder according to any one of claims 1 to 3, wherein the target disorder is major depression disorder, bipolar disorder, dysthymic disorder, cyclothymic disorder, depression disorder due to withdrawal from dependence-producing drugs, cognitive impairment due to aging, panic disorder, agoraphobia, social phobia, obsessive-compulsive disorder, posttraumatic stress disorder, acute stress disorder, or generalized anxiety disorder.

**5.** A method for curing or preventing mood disorder or anxiety disorder comprising administering an effective amount of an isoquinoline derivative represented by formula (I) or a physiologically acceptable acid addition salt thereof:

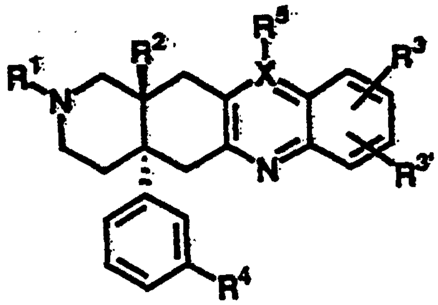

(I)

wherein $R^1$ is hydrogen, $C_1$-$C_5$ alkyl, $C_4$-$C_7$ cycloalkylalkyl, $C_5$-$C_7$ cycloalkenylalkyl, $C_7$-$C_{14}$ aralkyl, $C_4$-$C_5$ trans-alkenyl, allyl, furan-2-ylalkyl ($C_1$-$C_5$) , thien-2-ylalkyl($C_1$-$C_5$), $C_1$-$C_5$ alkanoyl, benzoyl, vinyloxycarbonyl, trichloroethoxycarbonyl, benzyloxycarbonyl, or $C_8$-$C_{14}$ arylalkanoyl; $R^2$ is hydrogen or $OR^6$ (wherein $R^6$ is hydrogen, $C_1$-$C_5$ alkyl, or $C_1$-$C_5$ alkanoyl); $R^3$ and $R^{3'}$ are, independently, $C_1$-$C_5$ alkyl, hydrogen, chlorine, fluorine, bromine, iodine, trifluoromethyl, cyano, hydroxy, $C_1$-$C_3$ alkoxycarbonyl, $C_1$-$C_3$ alkylcarbonylamino, $C_1$-$C_5$ alkoxy, nitro, amino, or $C_1$-$C_3$ alkylamino; $R^4$ is hydrogen, hydroxy, $C_1$-$C_3$ alkoxy, benzyloxy, $C_1$-$C_5$ alkanoyloxy, or halogen; X is nitrogen or carbon; $R^5$ exists only when X is carbon and is $C_1$-$C_5$ alkyl, hydrogen, chlorine, fluorine, bromine, iodine, trifluoromethyl, cyano, hydroxy, $C_1$-$C_3$ alkoxycarbonyl, $C_1$-$C_3$ alkylcarbonylamino, $C_1$-$C_5$ alkoxy, nitro, amino, or $C_1$-$C_3$ alkylamino.

**6.** The method for curing or preventing mood disorder or anxiety disorder according to claim 5, wherein, in formula (I), $R^1$ is $C_1$-$C_5$ alkyl, $C_4$-$C_7$ cycloalkylalkyl, $C_5$-$C_7$ cycloalkenylalkyl, $C_7$-$C_{14}$ aralkyl, $C_4$-$C_5$ trans-alkenyl, allyl, furan-2-ylalkyl($C_1$-$C_5$), or thien-2-ylalkyl($C_1$-$C_5$); $R^3$ and $R^{3'}$ are, independently, $C_1$-$C_5$ alkyl, hydrogen, chlorine, fluorine, bromine, iodine, hydroxy, $C_1$-$C_5$ alkoxy, nitro, amino, $C_1$-$C_3$ alkylamino, or $C_2$-$C_6$ dialkylamino; $R^4$ is hydrogen, hydroxy, $C_1$-$C_3$ alkoxy, or $C_1$-$C_5$ alkanoyloxy; X is carbon, and $R^5$ is $C_1$-$C_5$ alkyl, hydrogen, chlorine, fluorine, bromine, iodine, hydroxy, $C_1$-$C_5$ alkoxy, nitro, amino, $C_1$-$C_3$ alkylamino, or $C_2$-$C_6$ dialkylamino.

**7.** The method for curing or preventing mood disorder or anxiety disorder according to claim 5, wherein, in formula (I), $R^1$ is hydrogen, methyl, ethyl, cyclopropylmethyl, allyl, phenethyl, furan-2-ylethyl, or thiophen-2-ylethyl; $R^2$ is hydrogen, hydroxy, methoxy, ethoxy, or acetoxy; $R^3$ and $R^{3'}$ are, independently, methyl, hydrogen, chlorine, fluorine, bromine, iodine, hydroxy, methoxy, nitro, amino, or dimethylamino; $R^4$ is hydrogen, hydroxy, methoxy, or acetoxy; X is carbon; and $R^5$ is methyl, hydrogen, chlorine, fluorine, bromine, iodine, hydroxy, methoxy, nitro, amino, or dimethylamino.

**8.** The method for curing or preventing mood disorder or anxiety disorder according to one of claims 5 to 7, wherein the target disorder is major depression disorder, bipolar disorder, dysthymic disorder, cyclothymic disorder, depression disorder due to withdrawal from dependence-producing drugs, cognitive impairment due to aging, panic disorder, agoraphobia, social phobia, obsessive-compulsive disorder, posttraumatic stress disorder, acute stress disorder, or generalized anxiety disorder.

**9.** A use of an isoquinoline derivative represented by formula (I) or a physiologically acceptable acid addition salt thereof for the manufacture of a medicament for curing or preventing mood disorder or anxiety disorder:

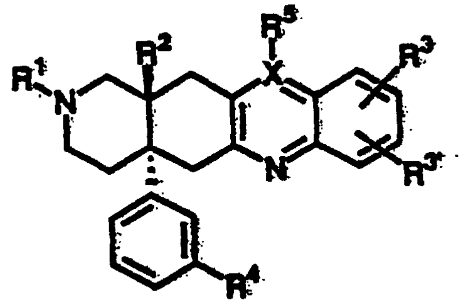

(I)

wherein $R^1$ is hydrogen, $C_1$-$C_5$, alkyl, $C_4$-$C_7$ cycloalkylalkyl, $C_5$-$C_7$ cycloalkenylalkyl, $C_7$-$C_{14}$ aralkyl, $C_4$-$C_5$ trans-alkenyl, allyl, furan-2-ylalkyl ($C_1$-$C_5$), thien-2-ylalkyl ($C_1$-$C_5$), $C_1$-$C_5$ alkanoyl, benzoyl, vinyloxycarbonyl, trichloroethoxycarbonyl, benzyloxycarbonyl, or $C_8$-$C_{14}$ arylalkanoyl; $R^2$ is hydrogen or $OR^6$ (wherein $R^6$ is hydrogen, $C_1$-$C_5$ alkyl, or $C_1$-$C_5$ alkanoyl); $R^3$ and $R^{3'}$ are, independently, $C_1$-$C_5$ alkyl, hydrogen, chlorine, fluorine, bromine, iodine, trifluoromethyl, cyano, hydroxy, $C_1$-$C_3$ alkoxycarbonyl, $C_1$-$C_3$ alkylcarbonylamino, $C_1$-$C_5$ alkoxy, nitro, amino, or $C_1$-$C_3$ alkylamino; $R^4$ is hydrogen, hydroxy, $C_1$-$C_3$ alkoxy, benzyloxy, $C_1$-$C_5$ alkanoyloxy, or halogen; X is nitrogen or carbon; $R^5$ exists only when X is carbon and is $C_1$-$C_5$ alkyl, hydrogen, chlorine, fluorine, bromine, iodine, trifluoromethyl, cyano, hydroxy, $C_1$-$C_3$ alkoxycarbonyl, $C_1$-$C_3$ alkylcarbonylamino, $C_1$-$C_5$ alkoxy, nitro, amino, or $C_1$-$C_3$ alkylamino.

**10.** The use of an isoquinoline derivative or a physiologically acceptable acid addition salt thereof for the manufacture of a medicament for curing or preventing mood disorder or anxiety disorder according to claim 9, wherein, in formula (I), $R^1$ is $C_1$-$C_5$ alkyl, $C_4$-$C_7$ cycloalkylalkyl, $C_5$-$C_7$ cycloalkenylalkyl, $C_7$-$C_{14}$ aralkyl, $C_4$-$C_5$ trans-alkenyl, allyl, furan-2-ylalkyl($C_1$-$C_5$), or thien-2-ylalkyl($C_1$-$C_5$); $R^3$ and $R^{3'}$ are, independently, $C_1$-$C_5$ alkyl, hydrogen, chlorine, fluorine, bromine, iodine, hydroxy, $C_1$-$C_5$ alkoxy, nitro, amino, $C_1$-$C_3$ alkylamino, or $C_2$-$C_6$ dialkylamino; $R^4$ is hydrogen, hydroxy, $C_1$-$C_3$ alkoxy, or $C_1$-$C_5$ alkanoyloxy; X is carbon, and $R^5$ is $C_1$-$C_5$ alkyl, hydrogen, chlorine, fluorine, bromine, iodine, hydroxy, $C_1$-$C_5$ alkoxy, nitro, amino, $C_1$-$C_3$ alkylamino, or $C_2$-$C_6$ dialkylamino.

**11.** The use of an isoquinoline derivative or a physiologically acceptable acid addition salt for the manufacture of a medicament for curing or preventing mood disorder or anxiety disorder according to claim 9, wherein, in formula (I), $R^1$ is hydrogen, methyl, ethyl, cyclopropylmethyl, allyl, phenethyl, furan-2-ylethyl, or thiophen-2-ylethyl; $R^2$ is hydrogen, hydroxy, methoxy, ethoxy, or acetoxy; $R^3$ and $R^{3'}$ are, independently, methyl, hydrogen, chlorine, fluorine, bromine, iodine, hydroxy, methoxy,. nitro, amino, or dimethylamino; $R^4$ is hydrogen, hydroxy, methoxy, or acetoxy; X is carbon; and $R^5$ is methyl, hydrogen, chlorine, fluorine, bromine, iodine, hydroxy, methoxy, nitro, amino, or dimethylamino.

**12.** The use of an isoquinoline derivative or a physiologically acceptable acid addition salt thereof for the manufacture of a medicament for curing or preventing mood disorder or anxiety disorder according to any one of claims 9 to 11, wherein the target disorder is major depression disorder, bipolar disorder, dysthymic disorder, cyclothymic disorder, depression disorder due to withdrawal from dependence-producing drugs, cognitive impairment due to aging, panic disorder, agoraphobia, social phobia, obsessive-compulsive disorder, posttraumatic stress disorder, acute stress disorder, or generalized anxiety disorder.

Fig. 1

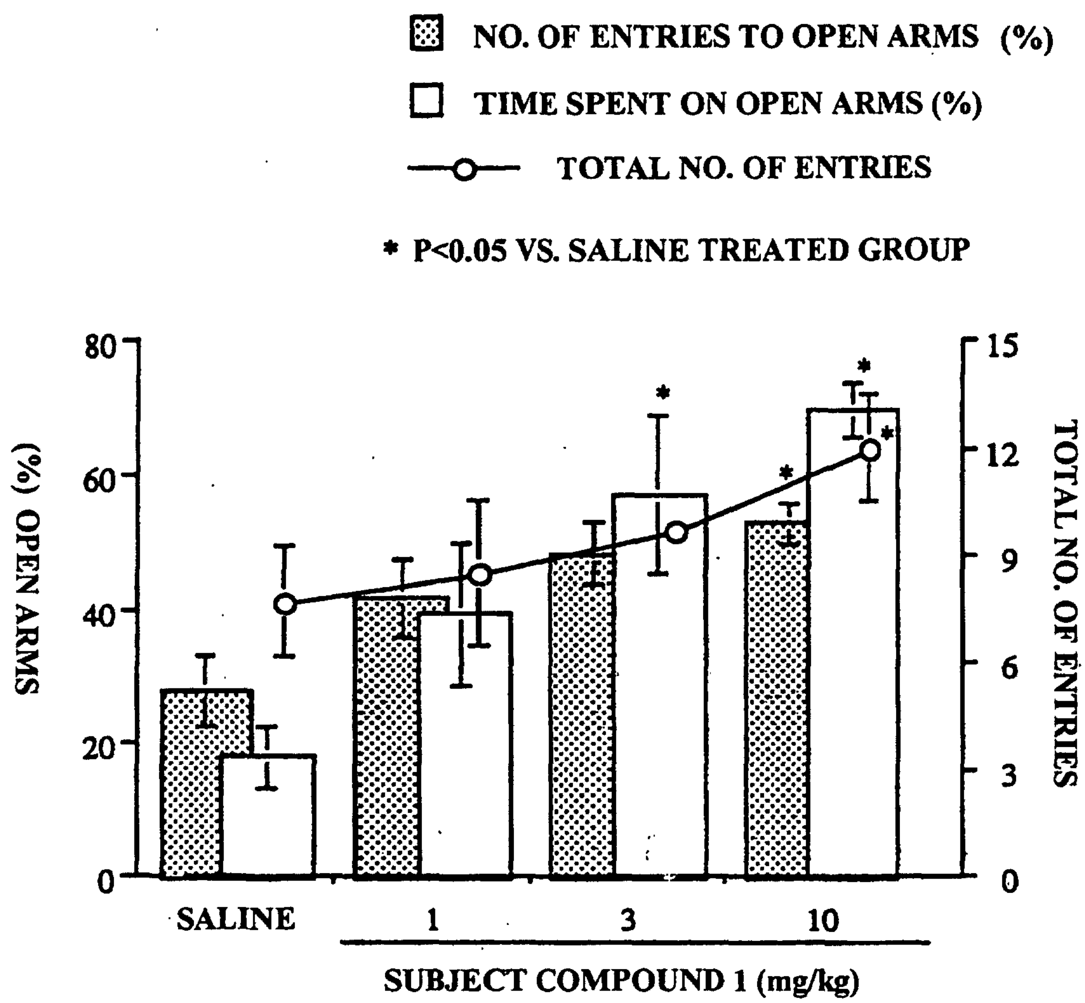
NO. OF ENTRIES TO OPEN ARMS (%)
TIME SPENT ON OPEN ARMS (%)
TOTAL NO. OF ENTRIES
* P<0.05 VS. SALINE TREATED GROUP
(%) OPEN ARMS
80
60
40
20
0
TOTAL NO. OF ENTRIES
15
12
9
6
3
0
SALINE
1
3
10
SUBJECT COMPOUND 1 (mg/kg)

Fig. 2

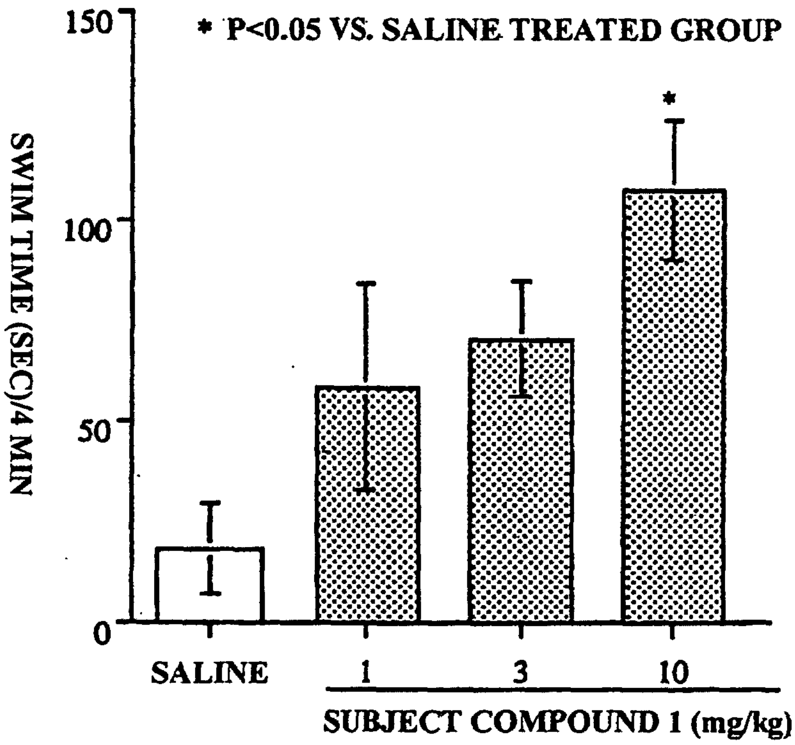
* P<0.05 VS. SALINE TREATED GROUP
SWIM TIME (SEC)/4 MIN
150
100
50
0
*
SALINE
1
3
10
SUBJECT COMPOUND 1 (mg/kg)

## INTERNATIONAL SEARCH REPORT

International application No.
PCT/JP01/05779

A. CLASSIFICATION OF SUBJECT MATTER
Int.Cl[7] A61K31/4745, 4985, C07D471/04, A61P25/24, 22, 18

According to International Patent Classification (IPC) or to both national classification and IPC

B. FIELDS SEARCHED

Minimum documentation searched (classification system followed by classification symbols)
Int.Cl[7] A61K31/435-549, C07D471/00-22, A61P25/00-36

Documentation searched other than minimum documentation to the extent that such documents are included in the fields searched

Electronic data base consulted during the international search (name of data base and, where practicable, search terms used)
CAPLUS(STN), REGISTRY(STN), WPI/L(DIALOG)

C. DOCUMENTS CONSIDERED TO BE RELEVANT

| Category* | Citation of document, with indication, where appropriate, of the relevant passages | Relevant to claim No. |
|---|---|---|
| PX | WO 01/40226 A1 (Toray Industries, Inc.), 07 June, 2001 (07.06.01), Claims (Family: none) | 1-4,9-12 |
| A | JP 4-275288 A (Toray Industries, Inc.), 30 September, 1992 (30.09.92), Claims (Family: none) | 1-4,9-12 |
| A | US 4939259 A (Eli Lilly & Co.), 03 July, 1990 (03.07.90), column 2, line 31 to column 3, line 49; column 13, lines 21 to 28 & EP 410641 A2 & JP 3-95182 A | 1-4,9-12 |

☐ Further documents are listed in the continuation of Box C. ☐ See patent family annex.

* Special categories of cited documents:
"A" document defining the general state of the art which is not considered to be of particular relevance
"E" earlier document but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| 11 September, 2001 (11.09.01) | 25 September, 2001 (25.09.01) |
| Name and mailing address of the ISA/ Japanese Patent Office | Authorized officer |
| Facsimile No. | Telephone No. |

Form PCT/ISA/210 (second sheet) (July 1992)

## INTERNATIONAL SEARCH REPORT

International application No.
PCT/JP01/05779

**Box I Observations where certain claims were found unsearchable (Continuation of item 1 of first sheet)**

This international search report has not been established in respect of certain claims under Article 17(2)(a) for the following reasons:

1. ☒ Claims Nos.: 5-8
because they relate to subject matter not required to be searched by this Authority, namely:

Claims 5-8 pertain to methods for treatment of the human body by therapy, and thus relate to subject matters which this International Searching Authority is not required, under the provisions of Article 17(2)(a)(i) of the PCT and Rule 39.1(iv) of the Regulations under the PCT, to search.

2. ☐ Claims Nos.:
because they relate to parts of the international application that do not comply with the prescribed requirements to such an extent that no meaningful international search can be carried out, specifically:

3. ☐ Claims Nos.:
because they are dependent claims and are not drafted in accordance with the second and third sentences of Rule 6.4(a).

**Box II Observations where unity of invention is lacking (Continuation of item 2 of first sheet)**

This International Searching Authority found multiple inventions in this international application, as follows:

1. ☐ As all required additional search fees were timely paid by the applicant, this international search report covers all searchable claims.

2. ☐ As all searchable claims could be searched without effort justifying an additional fee, this Authority did not invite payment of any additional fee.

3. ☐ As only some of the required additional search fees were timely paid by the applicant, this international search report covers only those claims for which fees were paid, specifically claims Nos.:

4. ☐ No required additional search fees were timely paid by the applicant. Consequently, this international search report is restricted to the invention first mentioned in the claims; it is covered by claims Nos.:

**Remark on Protest** ☐ The additional search fees were accompanied by the applicant's protest.
☐ No protest accompanied the payment of additional search fees.

Form PCT/ISA/210 (continuation of first sheet (1)) (July 1992)